# EUROPÄISCHE PATENTSCHRIFT

(11) **EP 3 520 686 B1**
(45) Veröffentlichungstag und Bekanntmachung des Hinweises auf die Patenterteilung: **29.01.2025**
(21) Anmeldenummer: 18154689.6
(22) Anmeldetag: 01.02.2018
(51) Int. Cl.: A61B 5/00, A61M 21/00

(54) **SYSTEM MIT VITALDATENSENSOR**
SYSTEM WITH VITAL DATA SENSOR
SYSTÈME À CAPTEUR DE DONNÉES VITALES

(43) Veröffentlichungstag der Anmeldung: 07.08.2019
(73) Patentinhaber: Vorwerk & Co. Interholding GmbH, 42275 Wuppertal (DE)
(72) Erfinder: Holz, Christian, 44137 Dortmund (DE); Mosebach, Andrej, 59425 Unna (DE)
(74) Vertreter: Gille Hrabal Partnerschaftsgesellschaft mbB Patentanwälte

(56) Entgegenhaltungen:
- EP-A1- 0 496 196
- WO-A1-2017/037250
- DE-A1- 102014 119 315
- US-A1- 2015 094 544
- US-A1- 2016 100 696

## Beschreibung

Die Erfindung betrifft ein System mit einer Auswertungseinheit und einem Sensor. Der Sensor kann einen Vitalparameter eines Lebewesens messen. Die Auswertungseinheit kann eine Auswertung auf Basis des gemessenen Vitalparameters durchführen.

Vitalparameter wie zum Beispiel Blutdruck geben Aufschluss über den aktuellen Zustand eines Lebewesens. Sogenannte Fitnesstracker zeichnen einen Vitalparameter auf und geben so beispielsweise einem Sportler Rückmeldung über seinen körperlichen Zustand. Beispiele für weitere solcher Systeme werden in den Druckschriften EP0496196A1, WO2017/037250A1, US2016/0100696A1, US2015/094544A1 und DE102014119315A1 beschrieben.

Es ist Aufgabe der Erfindung, ein weiterentwickeltes System bereitzustellen, das eine erweiterte Funktionalität im Zusammenhang mit anderen Geräten ermöglicht.

Zur Lösung der Aufgabe dienen ein System gemäß dem Hauptanspruch und eine Verwendung, ein Verfahren sowie ein Computerprogrammprodukt gemäß den Nebenansprüchen. Vorteilhafte Ausführungsformen ergeben sich aus den Unteransprüchen.

Zur Lösung der Aufgabe dient ein System mit einer Auswertungseinheit und einem Sensor. Der Sensor kann einen Vitalparameter eines Lebewesens messen. Die Auswertungseinheit kann eine Auswertung auf Basis des gemessenen Vitalparameters durchführen. Die Auswertungseinheit ist so beschaffen, dass die Auswertungseinheit in Abhängigkeit von einem Ergebnis der Auswertung einem externen Gerät, nämlich einer Küchenmaschine, , ein Kommandosignal zusenden kann, so dass das externe Gerät anhand des Kommandosignals eine Aktion ausführt. Der Benutzer kann dadurch Zeit einsparen und ein besonders hoher Bedienkomfort realisiert werden.

Ein Vitalparameter bezieht sich auf einen Organismus eines lebendigen Lebewesens und kann durch eine Maßzahl angegeben werden. In der Regel beschreibt ein Vitalparameter eine Grundfunktion bzw. Vitalfunktion des Lebewesens. Der Vitalparameter, d.h. die Maßzahl, kann mithilfe des Sensors ermittelt werden. Beispiele für Vitalparameter sind Körpertemperatur, Herzfrequenz, Atemfrequenz oder Blutdruck. Das Lebewesen ist ein Mensch.

Das System, durch das ein externes Gerät auf Basis eines gemessenen Vitalparameters eine Aktion ausführen kann, ermöglicht eine erweiterte Funktionalität im Zusammenhang mit externen Geräten, was nachfolgend anhand einiger Beispiele erläutert wird.

Das externe Gerät kann in einer nicht von den beiliegenden Ansprüchen umfassten Ausführungsform ein Haustürschloss, ein Rollladen- und/oder ein Smart-Home-Server sein. In einer Ausgestaltung ist das aktionsauslösende Ergebnis ein Einschlaf-Ereignis des Lebewesens. Das externe Gerät kann somit zeitlich unmittelbar nach dem Einschlaf-Ereignis, d.h. Einschlafzeitpunkt, eine Aktion ausführen oder einen bestimmten Betriebszustand einnehmen. Beispielsweise kann das Haustürschloss automatisch verriegelt, Rollladen und/oder Fenster automatisch geschlossen werden. In einer Ausgestaltung ist das aktionsauslösende Ergebnis ein Aufwach-Ereignis des Lebewesens. Beispielsweise kann dann die Heizung insbesondere im Badezimmer unmittelbar nach dem Aufwachen des Lebewesens aktiviert werden, so dass das Lebewesen, also beispielsweise eine Person, nach dem Aufwachen ein vorgewärmtes Badezimmer betreten kann.

In einer nicht von den beigefügten Ansprüchen umfassten Ausführungsform kann das Lebewesen ein Tier oder Haustier sein. Der Besitzer des Tiers oder Haustiers kann zeitlich unmittelbar nach dem Aufwachen des Tiers oder Haustiers benachrichtigt werden, um beispielsweise Türen und Fenster zu schließen. Alternativ oder ergänzend kann der Smart-Home-Server das externe Gerät sein, welches durch das Kommandosignal so angesteuert wird, dass alle Türen und Fenster geschlossen werden. Beispielsweise kann so ein Verletzen einer Katze an einem gekippten Fenster vermieden werden.

Wenn das externe Gerät der Smart-Home-Server ist, kann die Raumtemperatur und/oder die Beleuchtung in Abhängigkeit von dem durch die Auswertung ermittelten Ergebnis, insbesondere dem aktionsauslösende Ergebnis "unterkühlt", "überhitzt", "Einschlaf-Ereignis" und/oder "Aufwach-Ereignis" verändert werden.

In einer Ausgestaltung kann die Raumtemperatur durch das Kommandosignal gezielt verstellt werden und zwar insbesondere in Abhängigkeit von dem gemessenen Vitalparameter, z.B. der Körpertemperatur. Bei dem Ergebnis "unterkühlt" wird dann automatisch die Raumtemperatur erhöht.

Es kann ferner ermöglicht werden, dass eine Person nach dem Aufstehen frisch gebackene Brötchen und/oder einen frischen Kaffee einer Küchenmaschine entnehmen kann, wenn die Person die Küche erreicht hat.

Erfindungsgemäß ist das aktionsauslösende Ereignis ein prognostizierter Aufwachzeitpunkt und/oder der Einschlafzeitpunkt. Es wird so ermöglicht, dass die Küchenmaschine in Abhängigkeit von dem prognostizierten Zeitpunkt eine Aktion ausführt und somit besonders viel Zeit für den Benutzer eingespart werden kann. Wenn beispielsweise das Lebewesen ein Säugling ist, kann durch das Prognostizieren des Aufwachzeitpunkts eine Küchenmaschine zur Zubereitung eines Babybreis derart frühzeitig aktiviert werden, dass die Milchflasche bzw. der Babybrei kurz vor oder zumindest zeitgleich oder ungefähr zeitgleich mit dem Aufwachen des Säuglings fertig zubereitet ist. Die Eltern können so Zeit sparen und länger schlafen. Alternativ oder ergänzend kann das externe Gerät ein Benachrichtigungsgerät für eine oder zwei Personen, also z.B. für ein oder beide Elternteile sein. In einer Ausgestaltung umfasst das System einen zusätzlichen Sensor und eine zusätzliche Steuerungseinheit für jeweils beide Personen. Auf Basis des jeweils gemessenen Vitalparameters, der insbesondere in Form eines Messwertes an die Auswertungseinheit übertragen wird, kann somit der aktuelle körperliche Zustand der Personen ermittelt werden.

In einer Ausgestaltung führt die Auswertungseinheit eine Auswertung aus, deren Ergebnis diejenige Person angibt, die anhand der gemessenen Vitalparameter geeignet oder unter allen Personen am meisten geeignet ist, benachrichtigt zu werden. Insbesondere umfasst ein Benachrichtigen ein Aufwecken einer zu benachrichtigen Person, wenn diese schläft. Beispielsweise ist eine Person für eine Benachrichtigung geeignet, wenn sich diese in einer Schlafphase befindet, die oberhalb einer Schwelle liegt. Diese Person schläft dann nicht tief, so dass ein Aufwecken vergleichsweise wenig belastend für die Person ist. Beispielsweise ist eine Person geeigneter als eine andere Person, wenn die Auswertung der gemessenen Vitalparameter ergibt, dass die Person weniger tief schläft als die andere Person.

In einer Ausgestaltung wird die zu benachrichtigende Person in einer Weise benachrichtigt, dass nur die benachrichtigte Person aus dem Schlaf geweckt wird, nicht jedoch eine unmittelbar daneben liegende andere Person. Dies kann beispielsweise durch einen Vibrationsalarmgeber umgesetzt werden, der einen Vibrationsalarm besonders leise zum Beispiel auf eine Hautoberfläche applizieren kann. Insgesamt kann so beispielsweise nur ein nicht tief schlafender Elternteil bei Ermitteln eines Aufwachens oder Prognostizieren eines Aufwachzeitpunkts eines Säuglings geweckt werden, um zu einem bereits durch das System aktivierten externen Gerät zur Milchflaschen- und/oder Babybreizubereitung zu laufen, die fertig zubereitete Milchfalsche bzw. Babybreiportion zu entnehmen und dem Säugling zu verabreichen. Die Schlafzeit der Eltern zusammengenommen kann so maximiert werden.

Wenn der Sensor einen Vitalparameter eines Lebewesens misst, ist insbesondere vorgesehen, dass der Sensor ein entsprechendes Sensorsignal an eine mit dem Sensor verbundene Steuerungseinheit übermittelt. Vorzugsweise ist der Sensor mit der Steuerungseinheit zum Übermitteln des Sensorsignals oder zum Datenaustausch mit der Steuerungseinheit verbunden. In einer Ausgestaltung sind der Sensor und die Steuerungseinheit in einer Sendevorrichtung integriert. In einer Ausgestaltung kann die Steuerungseinheit eine Signalverarbeitung des Sensorsignals durchführen, d.h. eine Signalumwandlung und/oder Signaländerung. Vorzugsweise kann die Steuerungseinheit eine Analog-Digital-Umwandlung und/oder eine Signaländerung durch einen Algorithmus durchführen. Ein Sensorsignal ist ein insbesondere analoges Signal, dessen Spannung, Stromstärke und/oder Frequenz mit dem gemessenen Vitalparameter, d.h. dessen Maßzahl, korreliert. In einer bevorzugten Ausgestaltung entspricht das Messsignal, das auf Basis des Sensorsignals erzeugt und der Auswertungseinheit bereitgestellt wird, der Maßzahl des Vitalparameters. In einer Ausgestaltung wird das Sensorsignal durch die Steuerungseinheit und/oder die Sendevorrichtung lediglich in Form des Messsignals an die Auswertungseinheit weitergeleitet. Insbesondere führt die Steuerungseinheit dann nur eine Analog-Digital-Umwandlung von einem analogen Sensorsignal in ein digitales Messsignal durch. Das Messsignal ist bevorzugt digital. In einer Ausgestaltung weisen die Steuerungseinheit und/oder die Sendevorrichtung eine Datenschnittstelle insbesondere zum Datenaustausch mit der Auswertungseinheit auf. Vorzugsweise ist die Datenschnittstelle zum drahtlosen Datenaustausch eingerichtet. Beispielsweise ist die Datenschnittstelle eine WLAN-Schnittstelle, Funkschnittstelle und/oder Bluetooth-Schnittstelle. Das Messsignal kann somit besonders zuverlässig und kabellos zu einer entfernten und/oder mobilen Auswertungseinheit gesendet werden.

In einer Ausgestaltung weist ein Smartphone oder Tablet-PC die Auswertungseinheit auf. Die Anzahl der Komponenten für den Benutzer kann so reduziert werden und eine besonders einfache Bedienung über beispielsweise eine App erfolgen. In einer alternativen oder ergänzenden Ausgestaltung weist das externe Gerät die Auswertungseinheit auf. Wenn das externe Gerät die Auswertungseinheit aufweist, erfolgt das Zusenden des Kommandosignals insbesondere über eine Datenleitung oder ein Kabel. Eine weniger zuverlässige drahtlose Schnittstelle kann dann vermieden werden. Insbesondere wird die Auswertungseinheit bereitgestellt, indem ein Programmcode in ein aus anderen Gründen bereits vorhandenes Gerät implementiert wird.

In einer Ausgestaltung weist die Steuerungseinheit die Auswertungseinheit auf. Die Anzahl der Komponenten für den Benutzer kann so reduziert werden. Wenn die Steuerungseinheit die Auswertungseinheit aufweist, kann der Datenaustausch zwischen der Steuerungseinheit und der Auswertungseinheit ohne eine drahtlose Schnittstelle, also über eine Verbindung mittels Kabel, erfolgen.

In einer Ausgestaltung umfasst die Auswertungseinheit einen Prozessor, einen Speicher und/oder einen Computer-Programm-Code. In einer Ausgestaltung umfasst die Steuerungseinheit einen Prozessor, einen Speicher und/oder einen Computer-Programm-Code. Computer-Programm-Code meint auf einem Speicher speicherbare Befehle. Ein Prozessor, ein Speicher und/oder ein Computer-Programm-Code können so konfiguriert sein, dass ein Verfahren mit mehreren Verfahrensschritten durchgeführt werden kann. Durch Verfahrensschritte kann beispielsweise eine Signalverarbeitung, die Auswertung, das Erzeugen eines Kommandosignals, das Zusenden eines Kommandosignals an das externe Gerät und/oder das Ausführen einer Aktion erfolgen.

Eine Auswertung auf Basis eines gemessenen Vitalparameters erfolgt allgemein mithilfe eines Algorithmus. Das Messsignal und/oder das Sensorsignal können dann als Eingangsgröße bzw. Eingangsgrößen für den Algorithmus dienen. Als Ausgangsgröße gibt der Algorithmus das Ergebnis aus, dass insbesondere vordefinierte Zustände und/oder bestimmte Zustandsänderungen repräsentiert. In einer Ausgestaltung kann das Ergebnis auch ein "Null-Ereignis" sein, d.h., es wurde kein vordefinierter Zustand oder keine bestimmte Zustandsänderung durch die Auswertung ermittelt. Das Ergebnis ist dann kein aktionsauslösendes Ergebnis. Das bedeutet, es wird dann kein Kommandosignal für ein externes Gerät erzeugt oder das Kommandosignal ist dann ebenfalls ein Leersignal, bei dem das externe Gerät keine festgelegte Aktion anhand des Kommandosignals ausführt.

In einer Ausgestaltung kann das Ergebnis "unterkühlt", "überhitzt", "Tiefschlafphase" und/oder "Schlafphase mit geringer Schlaftiefe" sein. Das Ergebnis repräsentiert dann einen vordefinierten Zustand des Lebewesens. Es ist damit ein aktionsauslösendes Ergebnis.

Erfindungsgemäß ist das Ergebnis ein "Aufwach-Ereignis", d.h. ein Wechsel von einem Schlafzustand in einen Wachzustand, oder ein "Einschlaf- Ereignis", d.h. ein Wechsel von einem Wachzustand in einen Schlafzustand. Das Ergebnis repräsentiert dann eine bestimmte Zustandsänderung des Lebewesens. Es ist damit ein aktionsauslösendes Ergebnis. In einer Ausgestaltung wird das Ergebnis in Form eines digitalen Codes ausgegeben, beispielsweise "0", "1", "2", "3" oder "4".

In einer Ausgestaltung umfasst das Kommandosignal eine Zuordnung zu einer von mehreren in dem externen Gerät hinterlegten Aktionen. In einer Ausgestaltung umfasst das Kommandosignal eine Zuordnung zu einem externen Gerät, so dass nur in dem zugeordneten externen Gerät anhand des Kommandosignals eine Aktion ausgeführt wird. Unterschiedliche Aktionen können so in Abhängigkeit von dem gemessenen Vitalparameter gezielt an einem oder mehreren externen Geräten ausgelöst werden.

Die Aktion in einem externen Gerät ist bevorzugt durch ein Programm definiert, das auf einem Speicher insbesondere des externen Geräts hinterlegt ist. Durch das Kommandosignal wird dann ein hinterlegtes Programm aktiviert. Es können mehrere Programme hinterlegt sein.

Alternativ oder ergänzend kann die Aktion selbst durch das Kommandosignal vorgegeben werden. Das Kommandosignal entspricht dann beispielsweise einem Ansteuersignal mit Steuerbefehlen für eine Steuerung des externen Gerätes, das diese Steuerbefehle umsetzt. Ein externes Gerät ist ein eigenständiges und/oder bestehendes Gerät des Benutzers. Das externe Gerät befindet sich an einer beliebigen Stelle und in einem beliebigen Abstand relativ zum Sensor, der den Vitalparameter des Lebewesens messen kann. Das externe Gerät umfasst allgemein keinen Sensor, der zum Messen eines Vitalparameters eines Lebewesens eingerichtet und für diese Anwendung vorgesehen ist.

In einer Ausführungsform sind der Sensor und eine Steuerungseinheit in einer Sendevorrichtung integriert. Der Vitalparameter der Person kann somit jederzeit gemessen und der Auswertungseinheit bereitgestellt werden, insbesondere drahtlos.

In einer Ausführungsform wird ein Sensorsignal des Sensors durch die Steuerungseinheit in ein Messsignal umgewandelt, das mit dem gemessenen Vitalparameter korreliert und der Auswertungseinheit bereitgestellt wird. Eine drahtlose Übertragung der Messung an die Auswertungseinheit kann so ermöglicht werden. Ferner kann bereits eine Signalverarbeitung zur Entlastung der Auswertungseinheit in der Sendevorrichtung erfolgen.

In einer Ausführungsform ist die Sendevorrichtung zum Tragen am Körper des Lebewesens vorgesehen und eingerichtet. Zum Tragen am Körper meint ein enges Tragen am Körper oder unmittelbar am Körper, derart, dass zumindest eine Bewegung des Körpers in der Region, an dem die Sendevorrichtung getragen wird, durch einen Sensor zuverlässig aufgezeichnet werden kann. Insbesondere ist eine Befestigungseinrichtung zum Anbringen der Sendevorrichtung am Körper vorgesehen.

In einer Ausführungsform ist die Sendevorrichtung in einem Armband, einem Fußband, einem Stirnband, einer Brille, einem Hörgerät oder einem Kopfhörer integriert. Die Integration in ein Armband oder Fußband ermöglicht einen hohen Tragekomfort bei gleichzeitig engem Anliegen an der Körperoberfläche. Ein Stirnband ermöglicht ein Messen an der Kopfhaut. Eine Brille, ein Hörgerät und Kopfhörer ermöglichen einen direkten Kontakt eines Sensors mit der Kopfhaut, ohne dabei vom Benutzer unangenehm wahrgenommen zu werden.

In einer Ausgestaltung sind mindestens zwei separate Empfangsgeräte und/oder Sensoren an nur einem Lebewesen vorgesehen. Ein besonders präzises Auswerten des Körperzustands kann so ermöglicht werden.

In einer Ausführungsform ist der Sensor ein Hautkontaktsensor zum Messen von elektrischen Spannungsschwankungen auf einer Hautoberfläche des Lebewesens, insbesondere am Kopf. Die Messsignale für ein Elektroenzephalogramm können so bereitgestellt werden. Bei der Zustandsänderung vom Schlafzustand in den Wachzustand ändert sich eine gemessene Frequenz der Spannungsschwankungen von Alpha-Wellen zu Beta-Wellen. Umgekehrt ändert sich eine gemessene Frequenz der Spannungsschwankungen bei der Zustandsänderung vom Wachzustand in den Schlafzustand von Beta-Wellen zu Alpha-Wellen. Alpha-Wellen bezeichnen Wellen mit einem Frequenzbereich zwischen 8 und 13 Hz. Beta-Welle bezeichnet eine Welle mit einem Frequenzbereich zwischen >13 und 30 Hz. Zustandsänderungen wie ein Aufwach-Ereignis und Einschlaf-Ereignis sowie Zustände wie Tiefschlafphase und Schlafphase mit geringer Schlaftiefe können somit zuverlässig ausgewertet und als Ergebnis ausgegeben werden.

In einer Ausgestaltung kann durch einen Hautkontaktsensor alternativ oder ergänzend die Körpertemperatur gemessen werden. Die Körpertemperatur ist ein Vitalparameter, der unter anderem beispielsweise mit dem Schlaf/Wach-Zyklus korreliert. Zustandsänderungen wie ein Aufwach-Ereignis und Einschlaf-Ereignis sowie Zustände wie "unterkühlt" und "überhitzt" können somit ausgewertet und als Ergebnis ausgegeben werden. Vorzugsweise weist ein Hautkontaktsensor eine hautverträgliche Anlagefläche auf. Gesundheitsrisiken können so reduziert werden.

In einer Ausführungsform ist die Auswertungseinheit so eingerichtet, dass für die Auswertung des gemessenen Vitalparameters ein Vergleich mit einem Schwellwert durchgeführt wird. Das Sensorsignal oder das Messsignal werden also mit einem Schwellwert verglichen. Insbesondere kann vor dem Vergleich des Sensorsignals oder des Messsignals mit dem Schwellwert eine Signaländerung mittels eines Signaländerungsalgorithmus der Auswertungseinheit erfolgen, um eine besonders zuverlässige Auswertung durchführen zu können.

In der Ausführungsform, die als Sensor einen Hautkontaktsensor zum Messen von elektrischen Spannungsschwankungen auf einer Hautoberfläche des Lebewesens insbesondere am Kopf vorsieht, beträgt in einer bevorzugten Ausgestaltung der Schwellwert 11 bis 15 HZ, so zum Beispiel 13 Hz. Wenn das Messsignal zunächst kleiner als der Schwellwert ist und davon ausgehend den Schwellwert erreicht, wird in einer Ausgestaltung ein Ergebnis ausgegeben, das dem Aufwach-Ereignis zugeordnet ist. Das Lebewesen ist also aufgewacht. Wenn dass Messsignal zunächst größer als der Schwellwert ist und davon ausgehend den Schwellwert erreicht, wird in einer Ausgestaltung ein Ergebnis ausgegeben, das dem Einschlaf-Ereignis zugeordnet ist. Das Lebewesen ist also eingeschlafen.

In einer Ausführungsform ist der Sensor ein Gyrometer. Ein Gyrometer dient beispielsweise dem Messen einer Drehbewegung. Durch das Messen der Drehbewegung kann ein Maß für die Aktivität des Lebewesens ermittelt werden, das mit einer Zustandsänderung, z.B. Aufwach-Ereignis, in Korrelation gebracht werden kann. Insbesondere wird eine Richtungsänderung einer Drehbewegung erfasst und/oder pro Zeitintervall von z.B. zehn Sekunden gemessen. Erfolgen beispielsweise mindestens sechs Richtungswechsel in einem Zehn-Sekunden-Zeitraum, so ist dies ein Hinweis auf einen Wachzustand. Gleichsam ist ein steiler Anstieg der Anzahl der Richtungswechsel innerhalb eines Zeitraums von beispielsweise zehn Minuten vor dem Aufwachen mit einer insbesondere näherungsweise konstanten Steigung über die Zeit festzustellen. Der Einsatz eines Gyrometers als Sensor ermöglicht so eine besonders zuverlässige Prognose des Aufwachzeitpunkts. Wenn das Messsignal kleiner als ein Schwellwert, z.B. sechs Richtungswechsel in einem Zehn-Sekunden-Zeitraum, ist und davon ausgehend der Schwellwert erreicht wird, ist in einer Ausgestaltung "Aufwach-Ereignis" das Ergebnis der Auswertung. Wenn das Messsignal größer als der oben genannte Schwellwert ist und davon ausgehend der Schwellwert erreicht wird, ist in einer alternativen oder ergänzenden Ausgestaltung "Einschlag-Ereignis" das Ergebnis der Auswertung. Alternativ oder ergänzend ist der Sensor ein Kraftsensor, ein Kraftaufnehmer, ein Piezosensor und/oder ein Dehnungsmessstreifen. Das Lebewesen ist Insbesondere ein Säugling,

in einer nicht von den beiliegenden Ansprüchen umfassten Ausführungsform kann der mindestens eine Sensor ein Feuchtigkeitssensor zum Detektieren einer eingenässten Windel und/oder Schweißabsonderung, eine Bewegungssensormatte zur Aktivitätsmessung, ein Geruchssensor insbesondere für Methan, ein Pulsmesser, ein Blutdruckmesser, ein Gehimstromsensor für EEG und/oder EKG, ein Sauerstoffmesssensor insbesondere zur Ermittlung des Schlafabschnitts, ein MRT-Gerät insbesondere zur Ermittlung eines Aufwachzeitpunkts, eine Wärmebildkamera, eine Nachtsichtkamera insbesondere zur Ermittlung charakteristischer Bewegungsabläufe, eine Kamera mit Farbauflösung insbesondere zur Zuordnung der Hautfarbe, ein Blutzuckerspiegelsensor, ein CO₂-Messgerät für Atemluft, ein Pupillengrößen-Messgerät, ein Blinzelfrequenz-Messgerät insbesondere zum Prognostizieren eines Einschlafzeitpunkts und/oder ein Atemfrequenz-Messgerät sein. In einer Ausgestaltung wird der Sensor oder werden die Sensoren an einem Schlafplatz des Lebewesens, in oder an einer Decke und/oder in oder an einem Schlafsack befestigt. Ein oder mehrere Sensoren können dazu dienen, eine Körperhaltung beispielsweise während eines Schlafes zu ermitteln. So unterscheidet das System in einer Ausgestaltung zwischen solchen Körperhaltungen, die bei Unterkühlungen eingenommen werden, und solchen Körperhaltungen, die bei Unterkühlungen nicht eingenommen werden. In Abhängigkeit davon wird beispielsweise eine Heizung so gesteuert, dass eine Unterkühlung vermieden wird. Umgekehrt werden in einer Ausgestaltung der Erfindung ermittelte Körperhaltungen während eines Schlafes dazu genutzt, um Überhitzungen zu erkennen und in Abhängigkeit davon eine Klimaanlage so zu steuern, dass dem Überhitzen entgegengewirkt wird.

Insbesondere können die Vitalparameter ein, zwei oder drei aus den folgenden sein: Körpertemperatur, Aktivität, Puls, Sauerstoffgehalt im Blut, Blutzuckerspiegel, Gehirnstrom, charakteristische Bewegungsabläufe, charakteristische Körperhaltungen, Schweißabsonderung, CO₂-Atemluftgehalt, Atemfrequenz, Pupillengröße und/oder Blinzelfrequenz.

In einer Ausführungsform sind mindestens zwei Sensoren für unterschiedliche Vitalparameter vorgesehen. Eine Zustandsänderung kann so durch Berücksichtigung von zwei unterschiedlichen Vitalparametern besonders zuverlässig ermittelt werden. Beispielsweise kann also eine Körperhaltung sowie eine Schweißabsonderung während eines Schlafs eines Lebewesens mithilfe von dafür geeigneten Sensoren überwacht und in Abhängigkeit davon eine Klimaanlage gesteuert werden, um ein für ein Schlafen angenehmes Temperaturklima zu schaffen.

In einer Ausgestaltung wird bei der Auswertung eine Umgebungsinformation zusätzlich berücksichtigt. Insbesondere ist die Umgebungsinformation das Wetter bzw. eine Wettervorhersage, Mondfasen-Kalender, ein Fahrplan für einen Bus, der Bahn, der Müllabfuhr und/oder einen Staubsaugerroboter. Vorzugsweise weist die Auswertungseinheit eine Internetschnittstelle auf, um sich mit einem Wetterdienst, einem Smart-Home-Server z.B. mit dem Fahrplan des Staubsaugerroboters und/oder einer öffentlichen Fahrplanauskunft für Bus, Bahn und Müllabfuhr zu verbinden. In einer Ausgestaltung sind ein oder mehrere Temperatursensoren zur Erfassung einer Raumtemperatur, ein Helligkeitssensor zur Erfassung einer Raumhelligkeit, ein Luftfeuchtigkeitssensor zur Erfassung einer Raumluftfeuchtigkeit und/oder ein Mikrofon zur Erfassung von Verkehrslärm, Umgebungsgeräuschen oder Personengeräuschen wie Reden oder Schnarchen vorgesehen.

In einer Ausführungsform weist die Auswertungseinheit einen maschinellen Lernalgorithmus für die Auswertung und/oder die Ermittlung des Kommandosignals auf. Ein maschineller Lernalgorithmus für die Auswertung ermöglicht, dass ein körperlicher Zustand oder eine Zustandsänderung besonders zuverlässig auf Basis des gemessenen Vitalparameters ermittelt werden kann. Ein maschineller Lernalgorithmus für die Ermittlung des Kommandosignals ermöglicht, dass das externe Gerät zu einer besonders geeigneten Aktion von mehreren hinterlegten Aktionen unter Berücksichtigung des Ergebnisses der Auswertung veranlasst werden kann. Insgesamt kann durch den Einsatz eines maschinellen Lernalgorithmus das System an die Präferenzen und Eigenheiten des Lebewesens und/oder Benutzer angepasst werden.

Ein maschineller Lernalgorithmus ordnet allgemein einer oder mehreren Eingangsgrößen eine Ausgangsgröße zu und gibt diese in der Regel aus. Die Ausgangsgröße kann das Ergebnis und/oder das Kommandosignal sein. Ein maschineller Lernalgorithmus wird insbesondere durch einen Programmcode oder Algorithmus gebildet. Insbesondere wird ein maschineller Lernalgorithmus durch eine Modellbildungsphase und einer anschließenden Identifikationsphase erzeugt, um schließlich in einer Anwendungsphase einen Zeitpunkt für das Eintreten einer Zustandsänderung prognostizieren zu können. Insbesondere erfolgt die Modellbildungsphase beim Hersteller. Die Identifikationsphase kann beim Hersteller und/oder beim Endnutzer erfolgen. Die Anwendungsphase erfolgt dann beim Endnutzer. Zu Testzwecken kann die Anwendungsphase beim Hersteller erfolgen. In der Modellbildungsphase wird ein mathematisches Modell, d.h. Gleichungssystem, zur Zuordnung eines oder mehrerer Eingangsgrößen zu einer Ausgangsgröße erstellt. Eine Korrelation von einem oder mehreren Vitalparametern mit einem Zustand oder einer Zustandsänderung wird dabei berücksichtigt, d.h. in dem mathematischen Gleichungssystem abgebildet. Vorzugsweise wird in der Modellbildungsphase ein dynamisches Modell und/oder Differentialgleichungssystem für die Zuordnung der Ausgangsgröße zu einer Eingangsgröße oder eine Kombination von Eingangsgrößen erstellt. In dem Modell bzw. dem Differentialgleichungssystem dienen die Messsignale eines festgelegten Sensors oder mehrerer festgelegter Sensoren als Eingangsgröße bzw. Eingangsgrößen und das Ergebnis und/oder das Kommandosignal als die Ausgangsgröße. In der Identifikationsphase wird dem maschinellen Lernalgorithmus eine Mehrzahl von Werte-Paaren mit jeweils einer Eingangsgröße und einer Ausgangsgröße bzw. mit jeweils mehreren Eingangsgrößen und einer Ausgangsgröße zugeführt. Der maschinelle Lernalgorithmus wird auf diese Weise optimiert und der Realität angepasst. Insbesondere erfolgt eine Optimierung von Konstanten in einem Differentialgleichungssystem des maschinellen Lernalgorithmus auf Basis der zugeführten Werte-Paare. Durch das Vorsehen einer Rückmeldungseinrichtung kann dem maschinellen Lernalgorithmus eine Ausgangsgröße durch den Endnutzer zugeführt werden, worauf noch später näher eingegangen wird.

In der Anwendungsphase wird der maschinelle Lernalgorithmus angewendet, um anhand der ausgewerteten Messsignale das Ergebnis und/oder das Kommandosignal zu ermitteln, auszuwählen und/oder zuzuordnen.

In einer Ausführungsform ist eine Rückmeldungseinrichtung vorgesehen, durch die ein Benutzer dem maschinellen Lernalgorithmus eine Rückmeldung geben kann. Durch das Vorsehen einer Rückmeldungseinrichtung kann dem maschinellen Lernalgorithmus eine Ausgangsgröße durch den Benutzer zugeführt werden. Insbesondere kann der Benutzer so eine Rückmeldung über den Zeitpunkt des Eintreten eines Ereignisses wie z.B. eine bestimmten Zustandsänderung wie z.B. "aufgewacht" oder auch eines definierten Zustands wie z.B. "unterkühlt" geben. Der maschinelle Lernalgorithmus ist dabei so konfiguriert, dass das Ereignis der Rückmeldung planmäßig ein aktionsauslösendes Ergebnis zur Folge hat oder idealerweise haben sollte. Der maschinelle Lernalgorithmus kann somit z.B. typische Aufwachzeiten eines bestimmten Säuglings "erlernen" und berücksichtigen.

In einer Ausgestaltung umfasst die Rückmeldungseinrichtung einen Knopf oder Schalter, um eine Rückmeldung über das Eintreten eines definierten Ereignisses zu melden. In einer Ausgestaltung wird die Rückmeldungseinrichtung durch eine App für ein Smartphone oder Tablet-PC realisiert, um ereignisspezifische Rückmeldungen eingeben zu können.

In einer Ausführungsform ist die Auswertungseinheit so konfiguriert, dass das Kommandosignal ein Aktivieren, Öffnen und/oder Entriegeln des externen Gerätes auslösen kann. In einer alternativen oder ergänzenden Ausführungsform ist die Auswertungseinheit so konfiguriert, dass das Kommandosignal ein Deaktivieren, Schließen und/oder Verriegeln auslösen kann. Die Aktion ist also ein Aktivieren, Öffnen, Entriegeln, Deaktivieren, Schließen und/oder Verriegeln. Ein Küchengerät kann z.B. zum Zubereiten einer Speise aktiviert, eine Haustür z.B. verriegelt und/oder ein Fenster z.B. geschlossen werden.

In einer Ausführungsform ist die Auswertungseinheit so konfiguriert, dass das Kommandosignal ein Ändern einer Einstellung des externen Gerätes auslösen kann. Die Aktion ist also ein Ändern einer Einstellung des externen Gerätes. Das externe Gerät kann so an den aktuellen körperlichen Zustand des Lebewesens angepasst werden. Beispielsweise kann so die eingestellte Soll- Raumtemperatur einer Klimaanlage, die insbesondere durch den Smart-Horne-Server gesteuert wird, an die Körpertemperatur des Lebewesens angepasst werden. Erfindungsgemäß kann die Küchenmaschine mit automatisch erzeugten Rezepten oder automatisch vorgeschlagenen Rezeptempfehlungen die Rezepte den gemessenen Vitalparameter einer Person als das Lebewesen anpassen. So wird bei einem zu hohen Körperfettanteil beispielsweise der Fettgehalt in Zutaten eines Rezeptes reduziert und/oder ein Rezept mit geringem Fett-Anteil vorgeschlagen. Bei Personen, die unter Diabetes leiden, kann der Blutzucker durch den Sensor gemessen und/oder ein Rezept an den aktuellen Blutzucker angepasst werden.

Das Ausführen einer Aktion bei einem Haushaltsgerät in Abhängigkeit von der Auswertung eines gemessenen Vitalparameters ermöglicht, dass das Haushaltsgerät eine an die Bedürfnisses des Benutzers angepasste Aktion ausführt, ohne dass der Benutzer selbst tätig zu werden braucht. Eine Speise kann beispielsweise anhand des gemessenen Vitalparameters automatisch und/oder automatisiert zubereitet werden.

Eine Küchenmaschine kann so angezeigte Rezepte automatisch an das Lebewesen anpassen.

Erfindungsgemäß ist das externe Gerät und die davon durch das Kommandosignal entsprechend auslösbare Aktion ein Küchengerät zum entsprechenden Zubereiten einer Speise.

Ein weiterer Aspekt der Erfindung betrifft eine Verwendung des Systems nach dem vorhergehend beschriebenen Aspekt der Erfindung zur Lösung der eingangs gestellten Aufgabe, wobei das Ergebnis der Auswertung ein Einschlaf-Ereignis und/oder Aufwach-Ereignis ist. Wenn eine Person vom Schlafzustand in den Wachzustand wechselt, zeigt also das Ergebnis der Auswertung anhand des gemessenen Vitalparameters planmäßig an, dass ein "Aufwach-Ereignis" oder "Einschlaf-Ereignis" eingetreten ist. Zum Beispiel kann dann eine Haustür durch das Kommandosignal gemäß einem hinterlegten Programm verriegelt oder entriegelt werden. Das Lebewesen ist insbesondere eine Person.

In einer Ausführungsform der vorgenannten Verwendung ist das Lebewesen ein Säugling und/oder ein Kleinkind. Kleinkinder bzw. Säuglinge, welche insbesondere per Flasche gestillt werden müssen, wachen nachts häufig auf und schreien bzw. weinen insbesondere wegen Hunger. Dies führt in der Regel dazu, dass beide Eltern aufwachen, ein Elternteil aufsteht, eine Flasche lauwarme Milch und/oder eine Speise zubereitet, den Säugling bzw. das Kleinkind beruhigt und die zubereitete Nahrung verabreicht. Sowohl der Säugling bzw. das Kleinkind als auch beide Elternteile werden dadurch nachts häufig mehrfach für einen längeren Zeitraum wach gehalten. Durch die Messung des Vitalparameters am Säugling bzw. Kleinkind zur Auswertung des Schlafverhaltens und die entsprechend zeitgerechte Aktivierung eines Küchengeräts zur Zubereitung einer Speise für den Säugling kann eine Zeitersparnis bei der Bereitstellung der Speise für den Säugling ermöglicht werden.

In einer Ausgestaltung kann ein Küchengerät z.B. am Vorabend mit Kindernahrung bestückt werden. In einer alternativen oder ergänzenden Ausgestaltung kann eine Milchflaschenzubereitungsmaschine oder Teemaschine z.B. mit Milchpulver bestückt werden. Durch das Kommandosignal kann dann zeitnah zum Aufwach-Ereignis die entsprechende Speise automatisiert z.B. durch Aufwärmen einer Milchflasche oder das Vermischen von Milchpulver und temperiertem Wasser zubereitet werden und/oder bis zur Entnahme auf einer definierten Temperatur gehalten werden. Somit kann die Speise unmittelbar nach dem Aufwachen des Kindes und der Eltern verabreicht und die Zeit der Zubereitung eingespart oder zumindest reduziert werden. Eine besonders wertvolle Zeitersparnis für die Speisenzubereitung in der Nacht und/oder eine reduzierte Keimbildung durch einen reduzierten Zeitraum des Warmhaltens der Speise können so erzielt werden.

Ein weiterer Aspekt der Erfindung betrifft ein Verfahren, insbesondere nach dem eingangs beschriebenen Aspekt der Erfindung, bei dem ein Sensor einen Vitalparameter eines Lebewesens misst und eine Auswertungseinheit eine Auswertung auf Basis des gemessenen Vitalparameters durchführt. Die Auswertungseinheit senden in Abhängigkeit von einem Ergebnis der Auswertung einem externen Gerät, insbesondere Haushaltsgerät, ein Kommandosignal zu. Das externe Gerät führt anhand des Kommandosignals eine Aktion aus. Das externe Gerät kann somit eine an die Bedürfnisse des Lebewesens angepasste Aktion ausführen, ohne dass das Lebewesen selbst tätig zu werden braucht. Eine automatische Steuerung eines externen Geräts insbesondere für ein Haustier oder Tier als das Lebewesen wird so ermöglicht. Weitere Ausführungsformen und Vorteile, die sich analog auch auf dieses Verfahren beziehen, sind im Zusammenhang mit dem eingangs beschriebenen Aspekt der Erfindung beschrieben.

Ein Aspekt der Offenbarung betrifft ein Computerprogrammprodukt. Das Computerprogrammprodukt umfasst Befehle, die bei der Ausführung des Programms durch einen Computer diesen veranlassen, die Schritte des Verfahrens gemäß dem vorhergehenden Aspekt der Erfindung auszuführen. Insbesondere ist der Computer die Auswertungseinheit. Die Merkmale, Ausführungsformen und Wirkungen des eingangs beschriebenen Systems zur Lösung der Aufgabe beziehen sich analog auch auf dieses Computerprogrammprodukt.

Nachfolgend werden Beispiele offenbart, die einem verbesserten Verständnis der Erfindung dienen.

Es zeigen:
- Figur 1:: Schematische Darstellung eines Systems, das anhand eines gemessenen Vitalparameters ein Kommandosignal an ein externes Gerät senden kann, welches anhand des Kommandosignals eine Aktion ausführen kann, wobei gemäß der vorliegenden Erfindung das externe Gerät eine Küchenmaschine ist;
- Figur 2:: Schematische Darstellung des Aufbaus eines Systems, das anhand eines gemessenen Vitalparameters ein Kommandosignal an ein externes Gerät senden kann, welches anhand des Kommandosignals eine Aktion ausführen kann;
- Figur 3:: Schematische Darstellung eines Diagramms mit der Frequenz von auf einer Hautoberfläche gemessener elektrischer Spannungsschwankungen über die Zeit;
- Figur 4:: Schematische Darstellung eines Diagramms, welches die Messsignale aus einer Messung einer Aktivität über die Zeit abbildet.

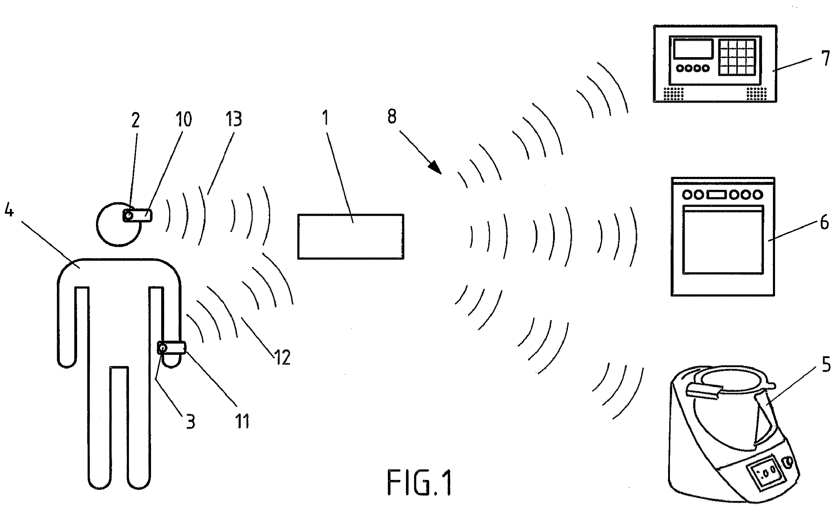

Die Figur 1 zeigt ein Lebewesen 4, das am Kopf eine Sendevorrichtung 10 mit einem Hautkontaktsensor 2 und/oder am Handgelenk eine weitere Sendevorrichtung 11 mit einem anderen Sensor, insbesondere ein Gyrometer 3, trägt. Die Sendevorrichtung 10 kann in einer Brille oder einem Stirnband integriert sein. Auch ein Aufkleben oder Befestigen mit einem Pflaster ist in einer Ausgestaltung vorgesehen. Das Lebewesen 4 ist ein menschliches Lebewesen bzw. eine Person und kann ein Säugling, ein Kleinkind, ein Mann oder eine Frau sein. Insbesondere dient der Hautkontaktsensor 2 dem Messen einer elektrischen Spannung auf der Hautoberfläche, so dass aus dem Sensorsignal Spannungsschwankungen ermittelt werden können. Alternativ oder ergänzend dient der Hautkontaktsensor 2 dem Messen der Körpertemperatur. Der andere Sensor bzw. das Gyrometer 3 ist in einem Armband integriert, derart, dass eine Bewegung des Handgelenks von dem anderen Sensor bzw. dem Gyrometer 3 erfasst wird.

Die mindestens eine Sendevorrichtung 10, 11 sendet bevorzugt drahtlos das mindestens eine Messsignal 12, 13 an eine Auswertungseinheit 1 zur Auswertung. In Abhängigkeit von einem Ergebnis der Auswertung erzeugt die Auswertungseinheit 1 ein Kommandosignal 8, das insbesondere drahtlos an mindestens ein externes Gerät 5, 6, 7 gesendet wird. Insbesondere sind wie dargestellt als externes Gerät eine Küchenmaschine 5vorgesehen. Vorzugsweise umfasst das Kommandosignal 8 eine Gerätzuordnungsinformation und eine Kommandoinformation. Die Kommandoinformation löst das Ausführen der Aktion durch ein bestimmtes externes Gerät 5, 6, 7 aus. Die Gerätzuordnungsinformation gibt das adressierte externe Gerät 5, 6, 7 an, für das die jeweilige Kommandoinformation vorgesehen ist. Vorzugsweise kann ein Kommandosignal 8 mehrere Sätze einer Gerätzuordnungsinformation und einer dazugehörigen Kommandoinformation umfassen. Mehrere externe Geräte 5, 6, 7 können so parallel anhand des Kommandosignals 8 jeweils eine Aktion ausführen.

Die Auswertungseinheit 1, die von der mindestens einen Sendevorrichtung 10, 11 oder der Steuerungseinheit 9 der Sendevorrichtung 10, 11 mindestens ein Messsignal 12, 13 empfängt, umfasst einen Prozessor 14 und einen Speicher 15. Insbesondere führt der Prozessor Schritte eines Verfahrens aus, die in Form eines Programms auf dem Speicher 15 gespeichert sind. Vorzugsweise umfasst das Programm einen maschinellen Lernalgorithmus. Die Auswertungseinheit 1 erzeugt in Abhängigkeit von einem Ergebnis der Auswertung ein Kommandosignal 8 und sendet das Kommandosignal 8 einem externen Gerät 5, 6, 7 zu, so dass das externe Gerät 5, 6, 7 anhand des Kommandosignals 8 eine Aktion ausführt, wie z.B. Licht einschalten und/oder ausschalten durch den Smart-Home-Server 7 oder eine Speise automatisiert zubereiten durch das Küchengerät 5 und/oder durch den Backofen 6.

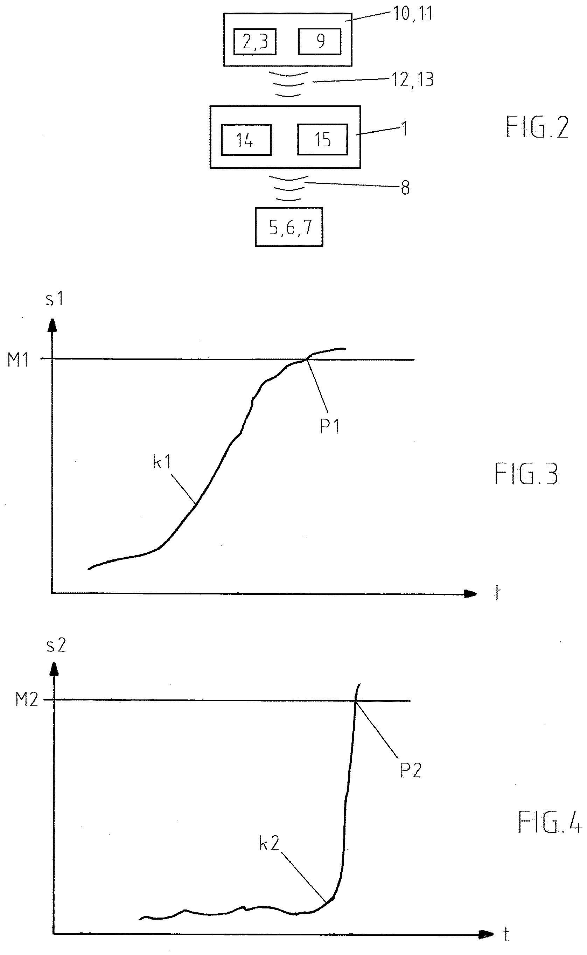

Die Figur 3 illustriert schematisch ein über die Zeit t aufgelöstes Diagramm, bei dem eine Messkurve k1 einen Vitalparameter s1 mit der Maßzahl einer Frequenz elektrischer Spannungsschwankungen an der Hautoberfläche am Kopf des Lebewesens 4 zeigt, die durch den Hautkontaktsensor 2 gemessen wurde. Insbesondere umfasst die Steuerungseinheit 9 und/oder die Auswertungseinheit 1 einen Algorithmus zur Ermittlung der Frequenz aus einem aufgezeichneten Verlauf der elektrischen Spannungsschwankungen, insbesondere aus einem Elektroenzephalogramm. Bei der Zustandsänderung vom Schlafzustand in den Wachzustand, also beim "Aufwach-Ereignis", ändert sich die Frequenz s1 von Alpha-Wellen zu Beta-Wellen. Umgekehrt ändert sich die Frequenz s1 bei der Zustandsänderung vom Wachzustand in den Schlafzustand, also beim "Einschlaf-Ereignis", von Beta-Wellen zu Alpha-Wellen. Ein Schwellwert M1 ist insbesondere bei einer Frequenz von 12, 13 oder 14 Hz vorgesehen. Die Auswertung umfasst einen Vergleich des Messsignals 11 oder der Messkurve k1 mit dem Schwellwert M1. Liegt das Messsignal unterhalb des Schwellwerts M1, ist "Wach-Zustand" das Ergebnis. Liegt das Messsignal oberhalb des Schwellwerts M1, ist "Schlaf-Zustand" das Ergebnis. Wird der Schwellwert M1 überschritten, ist "Aufwach-Ereignis" das Ergebnis. Wird der Schwellwert M1 unterschritten, ist "Einschlaf-Ereignis" das Ergebnis. In Figur 3 erfolgt ein solches Überschreiten im Schnittpunkt P1 der Messkurve k1 mit dem Schwellwert M1.

Erfindungsgemäß ist vorgesehen, anhand einer Messkurve aus Messsignalen 11, 12 einen Schnittpunkt mit einem Schwellwert durch Extrapolation der Messkurve zu prognostizieren.

Als Ergebnis wird dann der prognostizierte Aufwachzeitpunkt und/oder Einschlafzeitpunkt ausgegeben. Eine besonders hohe Zeitersparnis kann so erzielt werden.

Insbesondere kann dies auf das Beispiel der Figur 3 mit der Messkurve k1, dem Schwellwert M1 und dem Schnittpunkt P1 und/oder auf das Beispiel der Figur 4 mit der Messkurve k2, dem Schwellwert M2 und dem Schnittpunkt P2 angewendet werden.

Die Figur 4 illustriert schematisch ein weiteres Beispiel für ein Diagramm, bei dem eine Messkurve k2 einen Vitalparameter s2 mit der Maßzahl einer Aktivität über die Zeit t abbildet. Insbesondere entspricht die Messkurve den Messsignalen, die auf Basis der Sensorsignale des Gyrometers 3 vorzugsweise am Handgelenk des Lebewesens 4 ermittelt wurden. Die Maßzahl der Aktivität entspricht dabei der Anzahl von Richtungswechseln innerhalb eines definierten Zeitraums, z.B. zehn Sekunden. Wird eine Schwelle M2, z.B. sechs Richtungswechsel in einem Zehn-Sekunden-Zeitraum, überschritten, so ist "Aufwach-Ereignis" das Ergebnis der Auswertung. In Figur 4 erfolgt ein solches Überschreiten in Schnittpunkt P2 der Messkurve k2 mit dem Schwellwert M2.

## Patentansprüche

1. System mit einer Auswertungseinheit (1), einem Sensor (2, 3) und einem externen Gerät, wobei der Sensor (2, 3) einen Vitalparameter eines Lebewesens (4) messen kann und die Auswertungseinheit (1) eine Auswertung auf Basis des gemessenen Vitalparameters durchführen kann, wobei die Auswertungseinheit (1) so beschaffen ist, dass die Auswertungseinheit (1) in Abhängigkeit von einem Ergebnis der Auswertung einem externen Gerät (5, 6, 7) ein Kommandosignal (8) zusenden kann, so dass das externe Gerät (5, 6, 7) anhand des Kommandosignals (8) eine Aktion ausführt,
wobei das externe Gerät (5, 6, 7) eine Küchenmaschine (5) ist; wobei das Lebewesen (4) eine Person ist, wobei die ausgeführte Aktion ein automatisches Erzeugen eines Rezepts oder ein automatisches Vorschlagen einer Rezeptempfehlung durch die Küchenmaschine (5) ist,
wobei das aktionsauslösende Ereignis ein Einschlaf-Ereignis oder Aufwach-Ereignis der Person ist; wobei die Auswertungseinheit (1) auf Basis des gemessenen Vitalparameters einen prognostizierten Aufwachzeitpunkt und/oder Einschlafzeitpunkt ermittelt, wobei anhand einer Messkurve aus Messsignalen (11, 12) ein Schnittpunkt mit einem Schwellwert durch Extrapolation der Messkurve prognostiziert wird und der Schnittpunkt der prognostizierte Aufwachzeitpunkt und/oder Einschlafzeitpunkt ist, und der Sensor (2, 3) ein Hautkontaktsensor oder ein Gyrometer ist.

2. System nach dem vorhergehenden Anspruch, **dadurch gekennzeichnet, dass** der Sensor (2, 3) und eine Steuerungseinheit (9) in einer Sendevorrichtung (10, 11) integriert sind und/oder ein Sensorsignal des Sensors (2, 3) durch die Steuerungseinheit (9) in ein Messsignal (12, 13) umgewandelt wird, das mit dem gemessenen Vitalparameter korreliert und der Auswertungseinheit (1) bereitgestellt wird.

3. System nach dem vorhergehenden Anspruch, **dadurch gekennzeichnet, dass** die Sendevorrichtung (10, 11) zum Tragen am Körper des Lebewesens (4) vorgesehen und eingerichtet ist und/oder in einem Armband, einem Fußband, einem Stirnband, einer Brille, einem Hörgerät oder einem Kopfhörer integriert ist.

4. System nach einem der vorhergehenden Ansprüche, **dadurch gekennzeichnet, dass** der Sensor ein Hautkontaktsensor (2) zum Messen von elektrischen Spannungsschwankungen auf einer Hautoberfläche des Lebewesens (4) ist.

5. System nach einem der vorhergehenden Ansprüche, **dadurch gekennzeichnet, dass** die Auswertungseinheit (1) so eingerichtet ist, dass für die Auswertung des gemessenen Vitalparameters ein Vergleich mit einem Schwellwert (M1, M2) durchgeführt wird.

6. System nach einem der vorhergehenden Ansprüche, **dadurch gekennzeichnet, dass** zwei Sensoren (2, 3) für unterschiedliche Vitalparameter vorgesehen sind.

7. System nach einem der vorhergehenden Ansprüche, **dadurch gekennzeichnet, dass** die Auswertungseinheit (1) einen maschinellen Lernalgorithmus für die Auswertung und/oder die Ermittlung des Kommandosignals (8) aufweist.

8. System nach dem vorhergehenden Anspruch, **dadurch gekennzeichnet, dass** eine Rückmeldungseinrichtung vorgesehen ist, durch die ein Benutzer dem maschinellen Lernalgorithmus eine Rückmeldung geben kann.

9. System nach einem der vorhergehenden Ansprüche, **dadurch gekennzeichnet, dass** die Auswertungseinheit (1) so konfiguriert ist, dass das Kommandosignal (8) ein Aktivieren und/oder ein Deaktivieren des externen Gerätes (5, 6, 7) auslösen kann.

10. System nach einem der vorhergehenden Ansprüche, **dadurch gekennzeichnet, dass** die Auswertungseinheit (1) so konfiguriert ist, dass das Kommandosignal (8) ein Ändern einer Einstellung des externen Gerätes (5, 6, 7) auslösen kann.

11. System nach einem der vorhergehenden Ansprüche, **dadurch gekennzeichnet, dass** das externe Gerät (5, 6, 7) vom System umfasst ist.

12. Verfahren, bei dem ein Sensor (2, 3) einen Vitalparameter eines Lebewesens (4) misst und eine Auswertungseinheit (1) eine Auswertung auf Basis des gemessenen Vitalparameters durchführt, wobei die Auswertungseinheit (1) in Abhängigkeit von einem Ergebnis der Auswertung einem externen Gerät (5, 6, 7) ein Kommandosignal (8) zusendet und das externe Gerät (5, 6, 7) anhand des Kommandosignals (8) eine Aktion ausführt,
wobei das externe Gerät (5, 6, 7) eine Küchenmaschine (5) ist; wobei das Lebewesen (4) eine Person ist, wobei die ausgeführte Aktion ein automatisches Erzeugen eines Rezepts oder ein automatisches Vorschlagen einer Rezeptempfehlung durch die Küchenmaschine (5) ist,
wobei das aktionsauslösende Ergebnis ein Einschlaf-Ereignis oder Aufwach-Ereignis der Person ist; wobei die Auswertungseinheit (1) auf Basis des gemessenen Vitalparameters einen prognostizierten Aufwachzeitpunkt und/oder Einschlafzeitpunkt ermittelt, wobei anhand einer Messkurve aus Messsignalen (11, 12) ein Schnittpunkt mit einem Schwellwert durch Extrapolation der Messkurve prognostiziert wird und der Schnittpunkt der prognostizierte Aufwachzeitpunkt und/oder Einschlafzeitpunkt ist, und der Sensor (2, 3) ein Hautkontaktsensor oder ein Gyrometer ist.

13. Computerprogrammprodukt, umfassend Befehle, die bewirken, dass das System des Anspruchs 1 die Verfahrensschritte nach Anspruch 12 ausführt.

## Claims

1. System comprising an evaluation unit (1), a sensor (2, 3), and an external device, wherein the sensor (2, 3) can measure a vital parameter of an organism (4) and the evaluation unit (1) allows to conduct an evaluation based on the measured vital parameter, wherein the evaluation unit (1) is configured such that the evaluation unit (1) can send a command signal (8) to an external device (5, 6, 7) in dependency of a result of the evaluation, so that the external device (5, 6, 7) carries out an action based on the command signal (8)
wherein the external device (5, 6, 7) is a kitchen appliance (5); wherein the organism (4) is a person, wherein the performed action is an automatic generation of a recipe or automatic suggestion of a recipe recommendation by the kitchen appliance (5),
wherein the action-triggering event is a fall-asleep event or a wake-up event of the person; wherein the evaluation unit (1) determines a predicted wake-up time and/or fall-asleep time on the basis of the measured vital parameter, wherein, based on a measurement curve having measurement signals (11, 12), an intersection point with a threshold value is predicted by extrapolating the measurement curve and the intersection point is the predicted wake-up time and/or fall asleep time, and the sensor (2, 3) is a skin contact sensor or a gyrometer.

2. The system of the preceding claim, **characterized in that** the sensor (2, 3) and a control unit (9) are integrated in a transmitting device (10, 11) and/or a sensor signal of the sensor (2, 3) is converted by the control unit (9) into a measurement signal (12, 13) which correlates with the measured vital parameter and is provided to the evaluation unit (1).

3. The system of the preceding claim, **characterized in that** the transmitting device (10, 11) is provided and arranged to be worn on the body of the organism (4) and/or is integrated in a wristband, a footband, a headband, glasses, a hearing aid or a headphone.

4. The system of one of the preceding claims, **characterized in that** the sensor is a skin contact sensor (2) for measuring electrical voltage fluctuations on a skin surface of the organism (4).

5. The system of one of the preceding claims, **characterized in that** the evaluation unit (1) is configured such that a comparison with a threshold value (M1, M2) is carried out for the evaluation of the measured vital parameter.

6. The system of one of the preceding claims, **characterized in that** two sensors (2, 3) for different vital parameters are provided.

7. The system of one of the preceding claims, **characterized in that** the evaluation unit (1) comprises a machine learning algorithm for the evaluation and/or determination of the command signal (8).

8. The system of the preceding claim, **characterized in that** a feedback unit is provided by which a user can give a feedback to the machine learning algorithm.

9. The system of one of the preceding claims, **characterized in that** the evaluation unit (1) is configured such that the command signal (8) can trigger an activation and/or a deactivation of the external device (5, 6, 7).

10. The system of one of the preceding claims, **characterized in that** the evaluation unit (1) is configured such that the command signal (8) can trigger a change of a setting of the external device (5, 6, 7).

11. The system of one of the preceding claims, **characterized in that** the external device (5, 6, 7) is included in the system.

12. Method in which a sensor (2, 3) measures a vital parameter of an organism (4) and an evaluation unit (1) conducts an evaluation based on the measured vital parameter, wherein the evaluation unit (1) sends a command signal (8) to an external device (5, 6, 7) in dependency of a result of the evaluation, and the external device (5, 6, 7) carries out an action based on the command signal (8),
wherein the external device (5, 6, 7) is a kitchen appliance (5); wherein the organism (4) is a person, wherein the performed action is an automatic generation of a recipe or automatic suggestion of a recipe recommendation by the kitchen appliance (5),
wherein the action-triggering event is a fall-asleep event or a wake-up event of the person; wherein the evaluation unit (1) determines a predicted wake-up time and/or fall-asleep time on the basis of the measured vital parameter, wherein, based on a measurement curve having measurement signals (11, 12), an intersection point with a threshold value is predicted by extrapolating the measurement curve and the intersection point is the predicted wake-up time and/or fall asleep time, and the sensor (2, 3) is a skin contact sensor or a gyrometer.

13. Computer program product comprising instructions which cause the system of claim 1 to perform the method claims according to claim 12.

## Revendications

1. Système comprenant une unité d'évaluation (1), un capteur (2, 3) et un appareil externe, dans lequel le capteur (2, 3) peut mesurer un paramètre vital d'un être vivant (4) et l'unité d'évaluation (1) peut effectuer une évaluation sur la base du paramètre vital mesuré, dans lequel l'unité d'évaluation (1) est conçue de telle sorte que l'unité d'évaluation (1) peut envoyer un signal de commande (8) à un appareil externe (5, 6, 7) en fonction d'un résultat de l'évaluation, de sorte que l'appareil externe (5, 6, 7) exécute une action sur la base du signal de commande (8),
dans lequel l'appareil externe (5, 6, 7) est une machine de cuisine (5) ; dans lequel l'être vivant (4) est une personne, dans lequel l'action exécutée est une génération automatique d'une recette ou une proposition automatique d'une recommandation de recette par la machine de cuisine (5),
dans lequel l'événement déclencheur d'action est un événement d'endormissement ou un événement de réveil de la personne ; dans lequel l'unité d'évaluation (1) détermine, sur la base du paramètre vital mesuré, un moment de réveil et/ou un moment d'endormissement pronostiqué, dans lequel un point d'intersection avec une valeur seuil est pronostiqué à l'aide d'une courbe de mesure constituée de signaux de mesure (131, 12) par extrapolation de la courbe de mesure, et le point d'intersection est le moment de réveil et/ou le moment d'endormissement pronostiqué, et le capteur (2, 3) est un capteur de contact avec la peau ou un gyromètre.

2. Système selon la revendication précédente, **caractérisé en ce que** le capteur (2, 3) et une unité de contrôle (9) sont intégrés dans un dispositif d'envoi (10, 11) et/ou un signal de capteur du capteur (2, 3) est converti par l'unité de contrôle (9) en un signal de mesure (12, 13) qui est corrélé avec le paramètre vital mesuré et mis à disposition de l'unité d'évaluation (1).

3. Système selon la revendication précédente, **caractérisé en ce que** le dispositif d'envoi (10, 11) est prévu et agencé pour être porté sur le corps de l'être vivant (4) et/ou est intégré dans un bracelet, un bracelet de pied, un bandeau frontal, des lunettes, un appareil auditif ou un casque d'écoute.

4. Système selon l'une des revendications précédentes, **caractérisé en ce que** le capteur est un capteur de contact avec la peau (2) pour mesurer des variations de tension électrique sur une surface de peau de l'être vivant (4).

5. Système selon l'une des revendications précédentes, **caractérisé en ce que** l'unité d'évaluation (1) est agencée de telle sorte que, pour l'évaluation du paramètre vital mesuré, une comparaison est effectuée avec une valeur seuil (M1, M2).

6. Système selon l'une des revendications précédentes, **caractérisé en ce que** deux capteurs (2, 3) sont prévus pour des paramètres vitaux différents.

7. Système selon l'une des revendications précédentes, **caractérisé en ce que** l'unité d'évaluation (1) présente un algorithme d'apprentissage machine pour l'évaluation et/ou la détermination du signal de commande (8).

8. Système selon la revendication précédente, **caractérisé en ce qu'**il est prévu un dispositif de réponse par lequel un utilisateur peut donner une réponse à l'algorithme d'apprentissage machine.

9. Système selon l'une des revendications précédentes, **caractérisé en ce que** l'unité d'évaluation (1) est configurée de sorte que le signal de commande (8) peut déclencher une activation et/ou une désactivation de l'appareil externe (5, 6, 7).

10. Système selon l'une des revendications précédentes, **caractérisé en ce que** l'unité d'évaluation (1) est configurée de sorte que le signal de commande (8) peut déclencher une modification d'un réglage de l'appareil externe (5, 6, 7).

11. Système selon l'une des revendications précédentes, **caractérisé en ce que** l'appareil externe (5, 6, 7) est compris dans le système.

12. Procédé dans lequel un capteur (2, 3) mesure un paramètre vital d'un être vivant (4) et une unité d'évaluation (1) effectue une évaluation sur la base du paramètre vital mesuré, dans lequel l'unité d'évaluation (1) envoie un signal de commande (8) à un appareil externe (5, 6, 7) en fonction d'un résultat de l'évaluation et l'appareil externe (5, 6, 7) exécute une action à l'aide du signal de commande (8),
dans lequel l'appareil externe (5, 6, 7) est une machine de cuisine (5) ; dans lequel l'être vivant (4) est une personne, dans lequel l'action exécutée est une génération automatique d'une recette ou une proposition automatique d'une recommandation de recette par la machine de cuisine (5),
dans lequel le résultat déclenchant l'action est un événement d'endormissement ou un événement de réveil de la personne ; dans lequel l'unité d'évaluation (1) détermine, sur la base du paramètre vital mesuré, un moment de réveil et/ou un moment d'endormissement pronostiqué, dans lequel un point d'intersection avec une valeur seuil est pronostiqué à l'aide d'une courbe de mesure constituée de signaux de mesure (11, 12) par extrapolation de la courbe de mesure, et le point d'intersection est le moment de réveil et/ou le moment d'endormissement pronostiqué, et le capteur (2, 3) est un capteur de contact avec la peau ou un gyromètre.

13. Produit de programme d'ordinateur comprenant des instructions qui amènent le système de la revendication 1 à exécuter les étapes de procédé selon la revendication 12.
